(19) **Europäisches Patentamt**

**European Patent Office**

**Office européen des brevets**

(11) **EP 1 343 024 A1**

(12) **EUROPEAN PATENT APPLICATION**
published in accordance with Art. 158(3) EPC

(43) Date of publication:
**10.09.2003 Bulletin 2003/37**

(51) Int Cl.⁷: **G01T 1/161**, G01T 1/24,
G01T 1/17

(21) Application number: **00977874.7**

(22) Date of filing: **22.11.2000**

(86) International application number:
**PCT/JP00/08250**

(87) International publication number:
**WO 02/042797 (30.05.2002 Gazette 2002/22)**

(84) Designated Contracting States:
**DE NL**
Designated Extension States:
**AL LT LV MK RO SI**

(71) Applicants:
• **Kabushiki Kaisha Toshiba**
  **Tokyo 105-8001 (JP)**
• **Integrated Detector & Electron**
  **Hovik 1322 (NO)**

(72) Inventors:
• **SUNDAL, Bjorn**
  **Oslo N-0361 (NO)**
• **MORI, Issei**
  **Nishinasunomachi, Nasu-gun, Tochigi 329- (JP)**
• **TAKAYAMA, Takuzo**
  **Otawara-shi, Tochigi 324-0058 (JP)**

(74) Representative: **Granleese, Rhian Jane**
  **Marks & Clerk,**
  **57-60 Lincoln's Inn Fields**
  **London WC2A 3LS (GB)**

(54) **RADIATION IMAGE DIAGNOSTIC SYSTEM AND RADIATION DETECTOR**

(57)   A radiation image diagnosis apparatus comprises a radiation detector (25) which has a plurality of detection elements each converting radiation into an electric signal, an ASIC (26) which processes the electric signals outputted from the detection elements, power sources (30, 31) which supply power to the ASIC (26), and a power source monitor (28) which monitors the power feed from the power sources to the ASIC (26) and which stops the power feed from the power sources (30, 31) to the ASIC (26) upon sensing any abnormality in the electric signal. When any abnormality has been sensed in the electric signal, the power feed from the power sources (30, 31) to the ASIC (26) is stopped, so that the ASIC (26) can be protected from destruction ascribable to latchup. Besides, data are corrected on the basis of the length of the stop time period of the power feed to the ASIC (26), so that degradation in an image attributed to the power feed stop can be compensated for.

FIG. 9

EP 1 343 024 A1

## Description

Technical Field

The present invention relates to a radiation image diagnosis apparatus which is furnished with a semiconductor detector.

Background Art

Radiation image diagnosis apparatuses of this type include an X-ray computer tomography apparatus which images an internal body tissue structure as an X-ray absorption coefficient map, a gamma camera which images the density distribution of a radioisotope (RI) given into the body, and so forth.

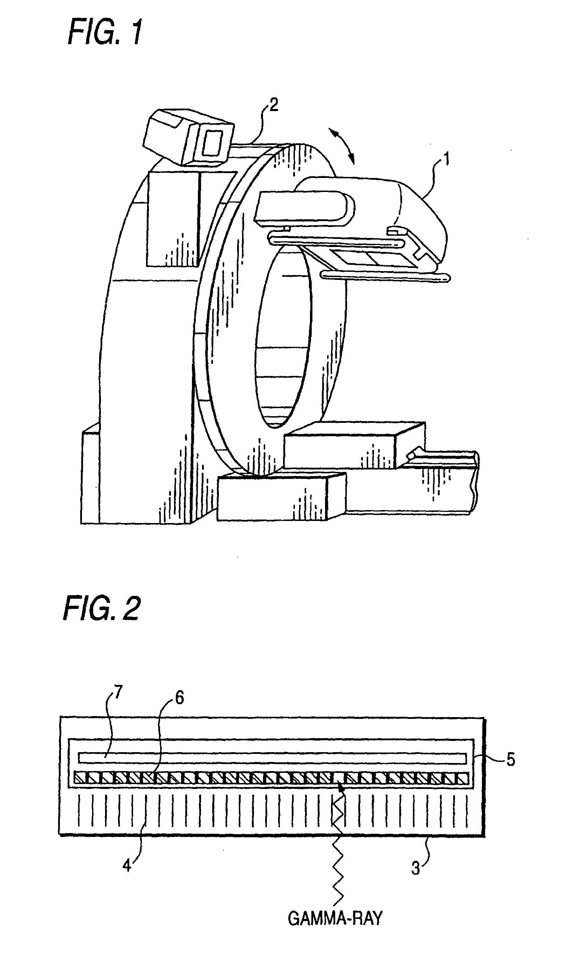

FIG. 1 shows the external appearance of a gamma camera. This example is a machine which serves both for planar radiography and for SPECT, and a detector of Anger type 1 which uses a plurality of photomultiplier tubes is rotatably supported by a stand 2. In recent years, there has been a trend to put into practical use a gamma camera based on a camera head which is furnished with a semiconductor detector having a large number of semiconductor detection elements (hereinbelow, simply called "detection elements") arrayed in the shape of a two-dimensional plane, instead of the Anger type detector 1. Although the semiconductor detector has also applications other than the gamma camera, the gamma camera shall be exemplified below.

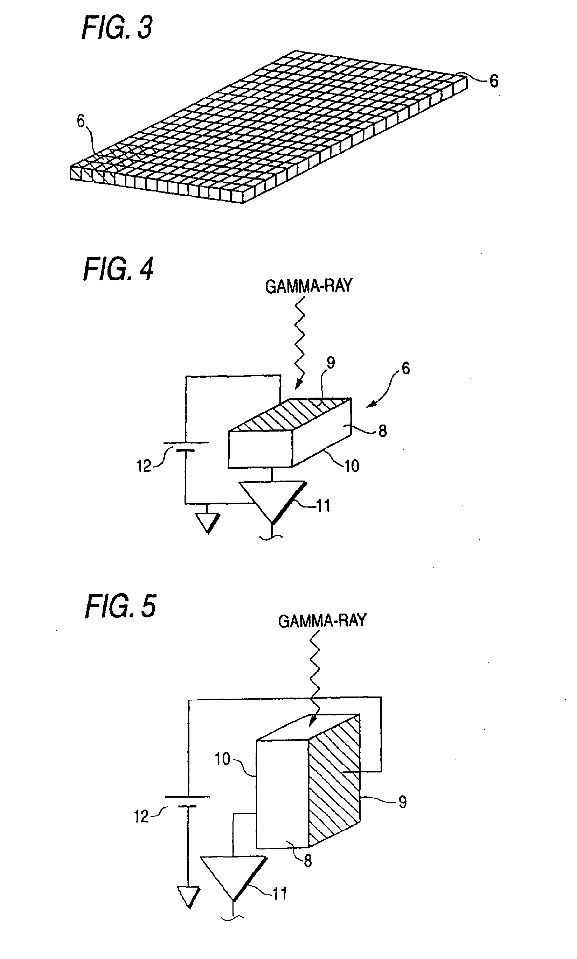

Shown in FIG. 2 is the sectional structure of a camera head. A collimator 4, and a semiconductor detector 5 arranged therebehind are accommodated in the camera head 3. The semiconductor detector 5 has detection elements 6. The detection elements 6 are actually arrayed in two dimensions in large numbers as shown in FIG. 3. Arranged behind the detection elements 6 is an ASIC 7 which has preamplifiers for processing the responses of the detection elements 6 to radiation entrances, readout circuits, etc.

As shown in FIG. 4, each detection element 6 is so constructed that electrodes 9, 10 are formed on both the surfaces of a semiconductor element 8 of CZT, CdTe or the like, and it stands ready for the radiation entrance in a state where a bias voltage is applied across the electrodes 9, 10 by a power source 12. When radiation enters the semiconductor element 8 in this state, charges migrate between the electrodes 9, 10, whereby a current flows between these electrodes 9, 10. The current signal is sent from the electrode (signal electrode) 10 and is amplified by a preamplifier 11, and the amplified signal is converted into a voltage signal, which is outputted. Incidentally, although the present-day mainstream is formed by the detection element of lateral electrode type shown in FIG. 4, there is also a detection element of vertical electrode type shown in FIG. 5.

Such detection elements 6 are arrayed in large numbers, whereby the wide-view detector in FIG. 3 is formed. This wide-view detector is realized by constructing one module out of, for example, 4 × 4 elements hatched in FIG. 3 and arraying a plurality of such modules.

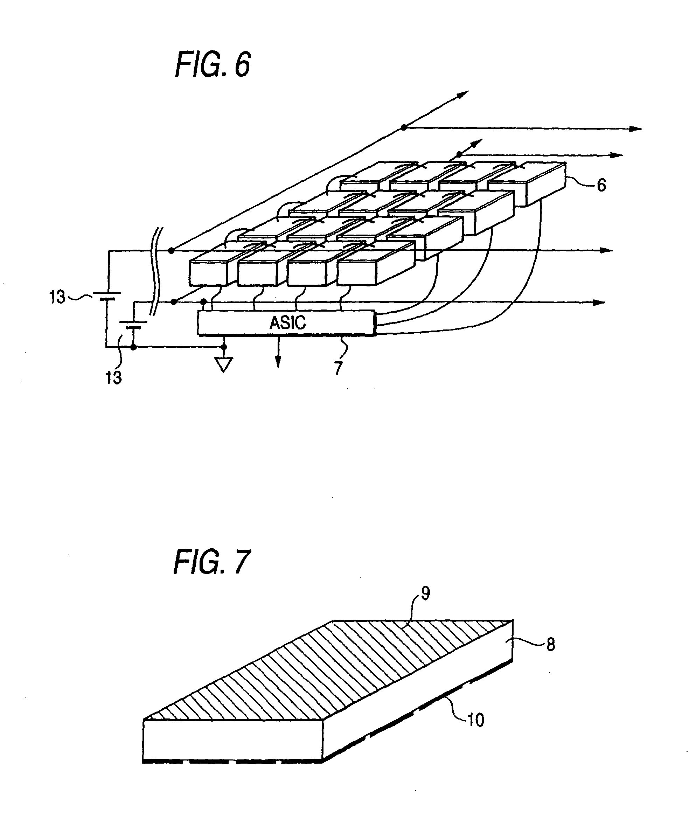

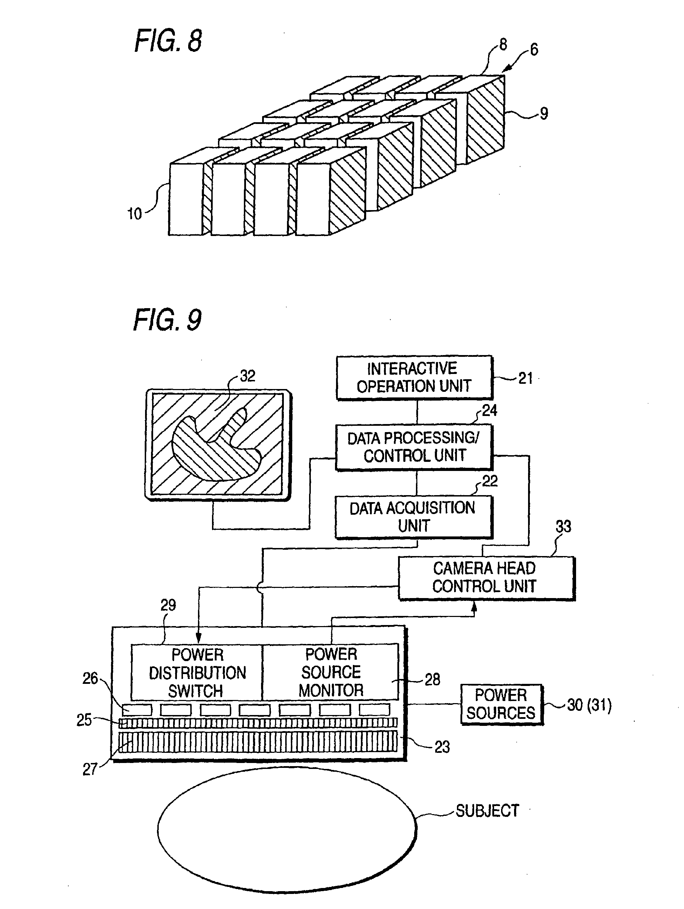

Shown in FIG. 6 are power sources and signal wiring lines for the single element module. Power source lines are extended also to the other modules. The preamplifier and the readout circuit are disposed every element, and circuits corresponding to a plurality of detection elements are constructed as one ASIC 7 in actual mounting. As the relationship between the element modules and the ASICs 7, it is common that one ASIC 7 corresponds to one module. However, this is not restrictive, but there are the relationships in which a plurality of ASICs 7 correspond to one module, and in which one ASIC 7 corresponds to a plurality of modules. Incidentally, the power sources 13 of the ASIC 7 are shown in FIG. 6, and they do not always have a single voltage, but they often have a plurality of voltages. For the brevity of explanation, however, an example of the single power source shall be described here. Besides, although the detection elements 6 are separated one by one in FIG. 6, there is also a module in which, as shown in FIG. 7, a semiconductor element 8 in the shape of a flat panel is used, and a bias applying electrode 9 is formed as a common electrode, while only signal electrodes 10 are individually formed. Of course, in case of the vertical electrode type, it is difficult to form a module corresponding to that of FIG. 7, so that separated detection elements 6 as shown in FIG. 8 are usually collected on a flat panel.

Meanwhile, the problem of latchup is always involved in such a semiconductor detector. An abnormal output (an output which is much larger than an ordinary response to radiation) is sometimes generated for a moment from the element side for any reason. Although the true cause for the abnormal output has not been made clear yet, this abnormal output is a phenomenon which inevitably occurs occasionally in the camera head having several thousand - several tens of thousand elements 6. A plurality of power sources are usually connected to the ASIC 7, and a structure in which a plurality of P-N junctions are joined in series (thyristor structure) is included anywhere though this depends also upon the sort of the ASIC 7. When the abnormally high input enters such an ASIC 7, it just triggers the thyristor, and even after the abnormal input has ended, an abnormal current continues to flow between the power sources or between the power source and the ground within the ASIC 7 (this is termed the "latchup") until the ASIC 7 is thermally destroyed. Even if the destruction is avoided, the ASIC 7 does not function during the flow of the abnormal current, and when the gamma camera is operated without noticing the malfunction, an image naturally becomes abnormal. Such an apparatus cannot endure practical use.

An object of the present invention is to protect an ASIC from destruction ascribable to latchup and to suppress an image abnormality, in a radiation image diagnosis apparatus furnished with a semiconductor detector.

Disclosure of the Invention

A radiation image diagnosis apparatus according to embodiments of the present invention comprises a radiation detector which has a plurality of detection elements each converting radiation into an electric signal; a bias power source which applies a bias voltage to the detection elements; a processing circuit which processes the electric signals outputted from the detection elements; a power source which supplies power to the processing circuit; a control unit which monitors at least one of the power feed from the bias power source to the detection elements and the power feed from the power supply means to the processing circuit, and which stops the power feed from the power source to the processing circuit when any abnormality has been sensed, and restarts the power feed from the power source to the processing circuit after a predetermined time period has lapsed since the stop of the power feed; and a correction unit which corrects data obtained by the detection elements, on the basis of a length of a stop time period of the power feed to the processing circuit.

Brief Description of the Drawings

FIG. 1 is a view showing the external appearance of a gamma camera which is an example of a radiation image diagnosis apparatus.
FIG. 2 is a sectional view showing the structure of a camera head in FIG. 1.
FIG. 3 is a view showing a detection element array in FIG. 2.
FIG. 4 is a detailed view of a detection element in FIG. 2.
FIG. 5 is a view showing another example (vertical type) of the detection element in FIG. 2.
FIG. 6 is a constructional view of one module which constitutes the detection element array in FIG. 2.
FIG. 7 is a view showing an example of construction in which bias electrodes are made common in the module in FIG. 6.
FIG. 8 is a constructional view of one module in the case of the vertical detection element in FIG. 5.
FIG. 9 is a functional block diagram of a radiation image diagnosis apparatus according to an embodiment of the present invention.
FIG. 10 is a diagram showing a power source monitor for monitoring the abnormalities of an element biasing power source and a power source for ASICs, and a power distribution switch for temporarily interrupting power feed to the ASIC in case of the abnormality, in this embodiment.
FIG. 11A is a diagram showing an example of construction of the biasing power source monitor in FIG. 10, and an output waveform in the case of the abnormality.
FIG. 11B is a diagram showing an example of construction of the ASIC power source monitor in FIG. 10, and an output waveform in the case of the abnormality.
FIG. 12 is a diagram showing another example of the power source monitor for monitoring the abnormalities of the element biasing power source and the power source for the ASICs in this embodiment.
FIG. 13 is a diagram showing the principle of a technique which corrects the lowering of an ASIC output signal attributed to the temporal interruption of power feed to the ASIC in this embodiment.
FIG. 14 is a diagram showing the principle of a technique which corrects for the decrease of projection lines attributed to the temporary interruption of power feed to the ASIC in case of coincidence PET in this embodiment.

Best Mode for Carrying Out the Invention

Now, a radiation image diagnosis apparatus according to the present invention will be described in conjunction with embodiments with reference to the drawings. Incidentally, radiation image diagnosis apparatuses include an X-ray computer tomography apparatus which images an internal body tissue structure as an X-ray absorption coefficient map, a gamma camera which images the density distribution of a radioisotope (RI) given into the body, and various other aspects. Here, the gamma camera shall be described as an example.

The essence of the present invention consists in that at least one of the element bias voltage (or current) of a detection element and the voltage (or current) of the power source (usually, there are a plurality of sorts of power sources, and among them, a line which has a high possibility of the occurrence of a thyristor phenomena is meant) of an ASIC is monitored, and that, when the monitored voltage has exhibited any abnormality as incurs latchup, power supply to the ASIC is quickly stopped once, while the power supply to the ASIC is automatically restarted after a predetermined time period has lapsed since the stop. At the point of time when the power supply to the ASIC is restarted, the voltage abnormality has often been eliminated. Further, the essence consists in that data during the power supply stop is corrected on the basis of the temporal length thereof, thereby to suppress any image abnormality attendant upon the power supply stop. The essences will be described in detail below.

FIG. 9 is a functional block diagram of the whole gamma camera system according to this embodiment. An interactive operation unit 21 is the man-machine interface between an operator and the system. A data acquisition unit 22 accumulates signals from a camera head 23, in a storage unit included in a data processing/control unit 24. The data acquisition unit 22 A/D-converts an analog signal from an electronic circuit (ASIC) 26 which subjects the output of a semiconductor detector 25 to amplification, current/ voltage conversion and other necessary preprocessing, so as to obtain the digital value of the energy value of each individual radiation entered. After the data acquisition unit 22 selects signals in accordance with a predetermined condition (such a condition that the signals fall within a predetermined energy window), it accumulates the signals in the storage device. The data processing/control unit 24 is a portion which chiefly functions to generate an image from the accumulated signals, and to control the data acquisition. The generated image is displayed on an image display unit 32.

The camera head 23 has the semiconductor detectors 25 arrayed in two dimensions, collimators 27, and the ASICs 26 which subject the outputs of the semiconductor detectors 25 to amplification and other necessary processing. These portions are the same as in the prior art, and in addition to the construction, the present invention includes a power source monitor 28 and a power distribution switch 29 as its features. Incidentally, reference numeral 30 designates a biasing power source 30, and reference numeral 31 an ASIC power source. Positions where these power sources are mounted are not especially restricted, but they may be either outside or inside the camera head 23.

The power source monitor 28 is a sensor which detects at least one of the element bias voltage (or current) of detection elements and the voltage (or current) of the power source of the ASICs, and the occurrence of any abnormality as incurs latchup is monitored on the basis of the output waveform of the power source monitor 28 by a camera head control unit 33. The power distribution switch 29 is a switch which is incorporated in a supply line extending from the ASIC power source 31 to the ASIC 26. When the output waveform of the power source monitor 28 has exhibited the abnormality, the camera head control unit 33 senses the abnormality and controls the power distribution switch 29 so as to interrupt power supply from the ASIC power source 31 to the ASIC 26. Besides, when the camera head control unit 33 has stopped the power feed to the ASIC 26 upon sensing the abnormality in the output waveform of the power source monitor 28, it notifies the operator of the power feed stop. Concretely, the camera head control unit 33 supplies the data processing/control unit 24 with information expressive of the stop of the power feed to the ASIC 26, in order that a message signifying the power feed stop may be displayed on the display unit 32, and/or that an alarm signifying the power feed stop may be sounded.

In this manner, the camera head control unit 33 stops the power feed to the ASIC 26 upon sensing the abnormality in the output waveform of the power source monitor 28, whereby it can prevent the latchup of the ASIC 26 from occurring.

Further, when a predetermined time period (a short time of, for example, several tens milliseconds to several hundred milliseconds) has lapsed since the point of time of the interruption of the power supply from the ASIC power source 31 to the ASIC 26, the camera head control unit 33 controls the power distribution switch 29 so as to restart the power supply from the ASIC power source 31 to the ASIC 26, in order to resume the radiographing (data acquisition). The reason why the time period for which the power supply from the ASIC power source 31 to the ASIC 26 is interrupted is set at several tens milliseconds to several hundred milliseconds, is that the set value is a time period which is required for the discharge of the smoothing capacitor of the power feed line. In a case, for example, where the time period for which the power supply is interrupted is set at several milliseconds or so, the discharge of the smoothing capacitor of the power feed line becomes insufficient.

Incidentally, positions where the power source monitor 28, power distribution switch 29 and camera head control unit 33 are mounted are not especially restricted, but they may be either outside or inside the camera head 23.

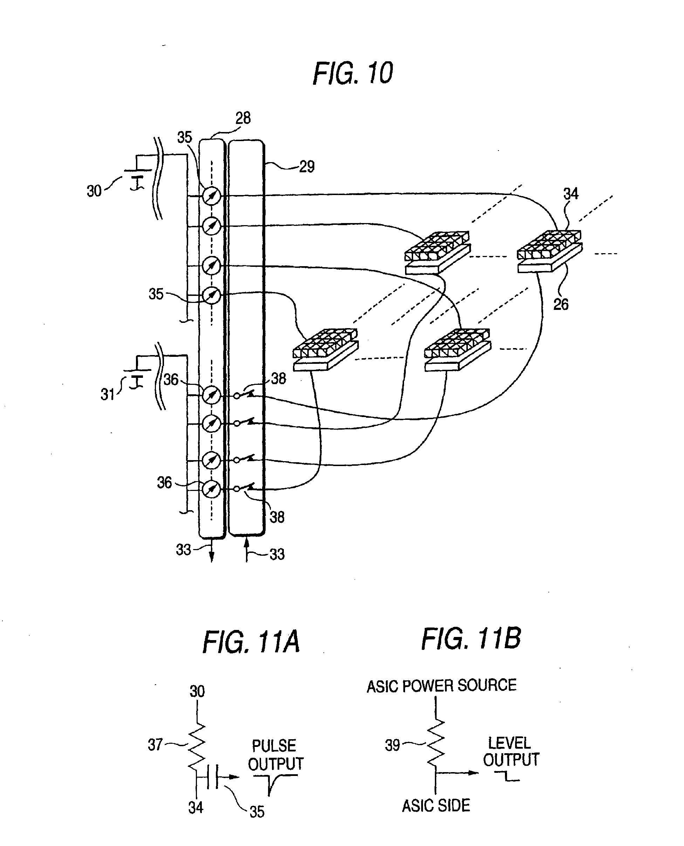

Practicable examples of the power source monitor 28 and the power distribution switch 29 are shown in FIG. 10. Here, the detection elements already stated are modularized, one module is set as one block, and the ASIC 26 corresponding to the single block is called "ASIC block". Besides, regarding the ASIC power source 31, only one line as to which the thyristor phenomenon is conspicuous is shown, and the other power source lines are not shown.

Next, the operation of this embodiment will be described.

(Case 1)

The actual circuit aspect of the power source monitor 28 for the element bias is constructed as shown in FIG. 11A by way of example. Usually, a current which flows through an element 34 is minute, and power is fed from the bias power source 30 via a high resistance 37 serving also as a protective resistance. The current is derived as a voltage signal through a capacitor 35. A line for the bias power supply is laid every element block 34, and the capacitor 35 is incorporated every power supply line in order to monitor the power supply of each individual element block 34. When an excess current has flowed through a certain element block 34 for a moment, a pulse voltage is generated from the capacitor 35 corresponding to the pertinent block 34, as shown in FIG. 11A. The pulse voltage is sensed by the camera head control unit 33, whereby a situation where the latchup may possibly be incurred can be grasped. Likewise to the bias power supply line, a line for the power supply to the ASIC block 26 is laid every ASIC block 26. When the pulse voltage has been sensed by the camera head control unit 33, a power distribution switch element 38 incorporated in the power supply line of the ASIC block 26 is opened (turned OFF) in order that the power supply to the ASIC block 26 corresponding to the pertinent element block 34 may be interrupted once. A time period for the opening may usually be a moment.

(Case 2)

Together with, or instead of the monitor 35 for the element bias, an ASIC power source monitor 36 is incorporated every ASIC power supply line as shown in FIG. 10. The actual aspect of the ASIC power source monitor 36 is a monitor for a voltage drop across a minute resistance 39 as shown in FIG. 11B. The camera head control unit 33 monitors the level output of the voltage drop monitor, and upon sensing the abnormality, it opens the power distribution switch element 38 incorporated in the power supply line of the ASIC block 26 having exhibited the abnormality, in order that the power supply to the pertinent ASIC block 26 may be interrupted once. A time period for the opening may usually be a short time.

In this manner, according to this embodiment, at least one of the element bias power supply and the ASIC power supply is monitored in block units, and even when either has exhibited any abnormal waveform, the power supply to the pertinent ASIC block is stopped. Thus, the latchup can be prevented from occurring. Further, the power supply is restarted after the lapse of the short time since the stop, but at this point of time, the waveform abnormality has ended in most cases, and the data acquisition can be directly resumed.

Incidentally, the power source monitor, the power distribution switch and the control unit therefor may well be assembled in the ASIC itself. With an appropriate ASIC design, even when the element output processing portion has incurred the latchup, such protective function portions can be prevented from suffering the damage of the latchup phenomenon.

Next, a modification to this embodiment will be described. This modification teaches an example of construction which is effective in a case where a distance from the power source monitor 35 to the camera head control unit 33 is physically long, so that wiring from the power source monitor 35 to the camera head control unit 33 is difficult in mounting.

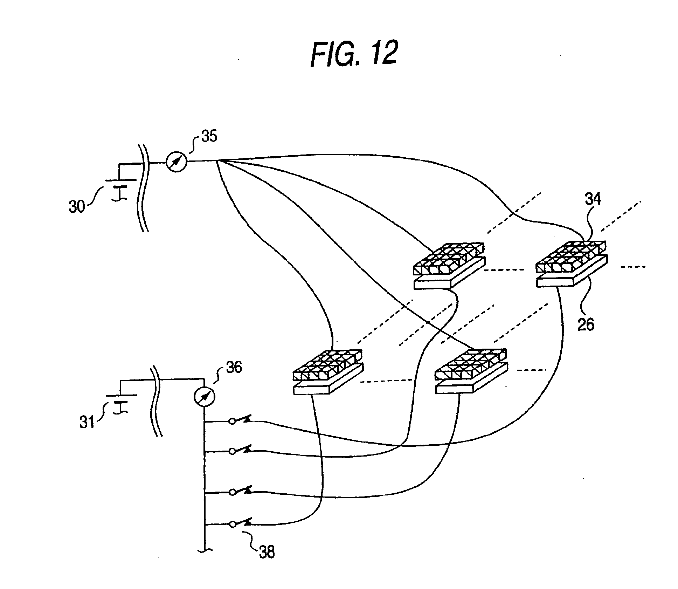

As shown in FIG. 12, this example is common to the foregoing aspect in the constructions of the power source monitors 35, 36 respectively shown in FIGS. 11A and 11B, but it is greatly different therefrom in the point that the power source monitors 35, 36 are shared by all the blocks 34, or that the power source monitors 35, 36 are shared in units of a plurality of neighboring blocks 34.

As stated before, when at least one of the bias power supply and the ASIC power supply has undergone the abnormality, the camera head control unit 33 simultaneously opens the distribution switches 38 leading to the plurality of ASIC blocks 26, thereby to prevent the latchup from occurring. In this manner, the ASIC power supply may well be stopped in units of the plurality of blocks by which the monitors 35, 36 are shared. Here, however, it is realized to stop the ASIC power supply in each individual block unit as in the foregoing embodiment.

The camera head control unit 33 supplies the data processing/control unit 24 with a control signal expressive of the occurrence of the abnormality. The data processing/control unit 24 accesses the storage device, and checks data which is being accumulated in the storage device from the data acquisition unit 22. Usually, no signal comes (a count value is zero or abnormally low) from the ASIC block 26 which is under the latchup. The data processing/control unit 24 specifies such an ASIC block 26, and sends information specifying the pertinent block 26, back to the camera head control unit 33. The camera head control unit 33 opens the power distribution switch 38 of the power source line leading to the pertinent block 26, for a short time in accordance with the information.

According to this example, it is permitted to decrease the numbers of the monitors 35, 36 and to simplify wiring from the monitors 35, 36 to the camera head control unit 33.

Next, there will be described the corrections of data during the power supply stop as are made by the data processing/control unit 24. As stated before, the camera head control unit 33 stops the ASIC power supply for the short time period from the point of time of the occurrence of the abnormality till the lapse of the predetermined time period. The camera head control unit 33 stores information items on the time of each power supply stop and the temporal length thereof in an internal memory or an external memory, and it occasionally sends the information items on the time of the stop and the temporal length $\Delta t$ to the data processing/control unit 24.

Usually, a data acquisition time period is divided into a plurality of sessions. Element outputs during one session (time period T) are integrated, and the result of the integration is handled as one measurement value. The time period $\Delta t$ of the power supply stop is set shorter than the session time period T. Accordingly, an ASIC output deviates (lowers) from its true value in accordance with the time period $\Delta t$ of the power supply stop. When an image is reconstructed in this state left intact (in any of planar radiography, SPECT and PET in case of a nuclear medical apparatus), degradation in the image is inevitable.

After the session time period T has lapsed, or after all measurement actions subsequent to this time period have been completed, the data processing/control unit 24 corrects data in the storage device, as stated below, and it supplies the corrected result for an image reconstruction computation (which the data processing/control unit 24 executes).

In case of the planar radiography and the SPECT, or non-coincidence PET with a collimator and the planar radiography, one session extends from the start of data acquisition to the end thereof. That is, it is a scanning time period T. In case of the SPECT and non-coincidence PET, the session is a measurement which is performed for a predetermined time period T in a state where the camera head 23 is at a certain turning angle. Since measurements are performed at the positions of many turning angles, one scanning is completed with a large number of sessions. The measurement values of the individual elements covered by the pertinent ASIC output are integrated till an intermediate time in the time period T, and the integrated value is denoted by X. This value is corrected in accordance with:

$$X' = X \cdot T / (T - \Delta t)$$

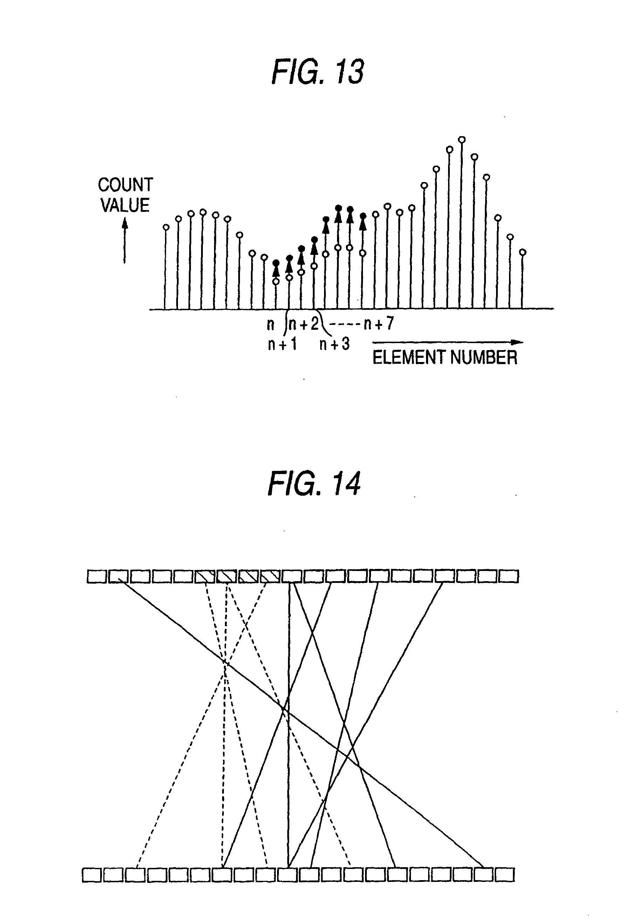

More specifically, as shown in FIG. 13, a count value actually measured is increased proportionally in correspondence with the time period $\Delta t$ of the power supply stop. In FIG. 13, a white circle mark represents the actual count value X, while a black circle mark represents the corrected value X'. Here, lowering in the count values as is attendant upon the temporary power supply stop is observed in one block which consists of the eight elements of element Nos. $\underline{n}$ through (n + 7), and the measurement values of these elements are corrected.

Here, in coincidence PET, a measurement at a certain turning angle is set as one session, the time period of which corresponds to the above time period T. Regarding gamma-rays detected by a plurality of opposing elements at an identical timing, a virtual line LOR (Line Of Response) connecting the elements having detected the gamma-rays is generated at the pre-stage of the image reconstruction computation (the generation is executed by the data processing/control unit 24), and it is used as projection data for the image reconstruction computation. Using data obtained till an intermediate time in the time period T, the LORs are generated for the data of the elements covered by the pertinent ASIC 26, similarly between these elements and the other elements. FIG. 14 shows an example in a system in which two camera heads 23 oppose to each other. Although the LORs are drawn in only one dimension, they actually extend in two dimensions. The example corresponds to a case where the pertinent elements are in the number of four. A plurality of LORs are indicated by a single line. The LORs in solid lines are directly used without changing their number, but the LORs in dotted lines, which are drawn between the problematic elements and the certain elements, are supplied for the image reconstruction after having their number X corrected as follows:

$$X' = X \cdot T/(T - \Delta t)$$

Owing to the above correction, a good image can be generated in spite of the existence of the stop time period $\Delta t$.

Incidentally, the ASIC which incurs the latchup phenomenon has been exemplified in the above. However, the present invention is not restricted to the ASIC or to the latchup, but it is applicable to the general circuits each of which has such a characteristic that, when the operation of a circuit for processing element outputs has become abnormal irrespective of the reason for the abnormality, it is resumed by disconnecting a power supply line once.

Besides, there has been described the example in which the data acquisition is continued after turning OFF the power distribution switch for only some of the ASIC blocks for the short time, and the acquired data are thereafter corrected. It is also allowed, however, that, when the turn-OFF of the power distribution switch has become necessary, some or all of the ASIC blocks are temporarily turned OFF, the data acquisition for the time period T stopped halfway is redone from the beginning.

In the above, the time period of the power supply stop as indicated by $\Delta t$ has been supplied for the correction of the acquired data. In actuality, however, there are time periods in which the normal operation is not immediately effected in spite of the restart of the power supply, for example, a time period in which the ASIC recovers from an abnormal heated state. The system can also discard ASIC outputs in the meantime, for the data acquisition in anticipation of the time periods. In such a case, this patent shall be understood with $\Delta t$ denoting the time period which is expended since the sensing of the abnormality till the restart of the data acquisition.

Besides, it has been described above that the power source lines leading to the ASICs, for which the abnormality is monitored, are the same as the power source lines for which the power distribution is turned ON/OFF. In some circuit designs, however, the ASICs can be protected and resumed in such a way that the line having undergone the abnormality is not turned OFF, but that another power source line (in this case, ground connection can also be regarded as one of the power source lines) is turned OFF. Even such a case shall also be naturally covered within the present invention.

Further, it has been described above that the element blocks and the ASIC blocks are in a one-to-one correspondence. However, although the one-to-one correspondence is desirable for Case 1, naturally the element and ASIC blocks may correspond either at N : 1 or at 1 : N in the other cases. Besides, it has been described above that a plurality of elements are supposed to be ordinarily modularized before juxtaposing the detectors, and that the module is set as the unitary block for the power source, the bias supply and the control. It is natural, however, that a plurality of modules may well be set as one block.

Besides, it has been described that the numbers of the individual power distribution switches and the individual power source monitors are at 1 : 1, but they may well be at 1 : N or N : 1. Besides, the power distribution switch and the power source monitor are disposed every block for the reason of circuit mounting, but one element may naturally be considered as one block in such a case where the size of each element is large and where the number of the elements is small.

Besides, the example in which the radiation detection elements are arrayed in two dimensions has been mentioned, but it is to be understood that the elements may well be arrayed in one dimension or three dimensions.

Although there has been exemplified the semiconductor detector application based on the photon counting system including gamma camera, the semiconductor detector is also usable in a continuous current mode as in X-ray CT or a roentgen apparatus. The drawings of the embodiment hold true mostly, and the description holds true mostly. A mere difference is that the acquired data is not the count value, but that it is a current magnitude within a predetermined time period or the A/D value of the quantity of accumulated charges, and the axis of ordinates in FIG. 13 may be changed from the count value to a measurement value.

The present invention is not restricted to the foregoing aspects of performance, but it can be variously modified and performed within a scope not departing from the purport thereof. Further, various stages are included in the aspects of performance, and various inventions can be derived by properly combining the plurality of disclosed constituents. By way of example, some constituents may be deleted from all the constituents indicated in the aspects of performance.

Industrial Applicability

As described above, the present invention is suited to provide a radiation image diagnosis apparatus which is furnished with a semiconductor detector.

**Claims**

1. A radiation image diagnosis apparatus, comprising:

   radiation detection means having a plurality of detection elements each of which converts radiation into an electric signal;
   a bias power source which applies a bias voltage to the detection elements;
   a processing circuit which processes the electric signals outputted from said detection elements;
   power supply means for supplying power to said processing circuit;
   monitor means for monitoring at least one of the power feed from said bias power source to said detection elements and the power feed from said power supply means to said processing circuit;
   control means for stopping said power feed from said power supply means to said processing circuit when said monitor means has sensed any abnormality; and
   correction means for correcting data obtained by said detection means, on the basis of a length of a stop time period of said power feed to said processing circuit.

2. A radiation image diagnosis apparatus as defined in claim 1, **characterized in that** said processing circuit is constructed of a plurality of processing circuit blocks, and that said control means stops said power feed every processing circuit block.

3. A radiation image diagnosis apparatus as defined in claim 2, **characterized in that** each of said processing blocks is disposed every detection element.

4. A radiation image diagnosis apparatus as defined in claim 1, **characterized in that** said control means restarts said power feed to said processing circuit after a predetermined time period has lapsed since the stop of said power feed to said processing circuit.

5. A radiation image diagnosis apparatus as defined in claim 4, **characterized in that** said power feed to said processing circuit is restarted by resuming a state where said power feed has been stopped.

6. A radiation image diagnosis apparatus, comprising:

   radiation detection means having a plurality of detection elements each of which converts radiation into an electric signal;
   a bias power source which applies a bias voltage to the detection elements;
   a processing circuit which has a plurality of processing circuit blocks each processing the electric signals outputted from said detection elements;
   power supply means for supplying power to said processing circuit;
   monitor means for monitoring at least one of the power feed from said bias power source to said detection elements and the power feed from said power supply means to said processing circuit; and

control means for stopping said power feed from said power supply means to the corresponding processing block of said processing circuit when said monitor means has sensed any abnormality.

7. A radiation image diagnosis apparatus as defined in claim 6, **characterized in that** said control means restarts said power feed to said processing block after a predetermined time period has lapsed since the stop of said power feed to said processing block.

8. A radiation image diagnosis apparatus, comprising:

   radiation detection means having a plurality of detection elements each of which converts radiation into an electric signal;
   a bias power source which applies a bias voltage to the detection elements;
   a processing circuit which processes the electric signals outputted from said detection elements;
   power supply means for supplying power to said processing circuit;
   monitor means for monitoring at least one of the power feed from said bias power source to said detection elements and the power feed from said power supply means to said processing circuit; and
   control means for stopping said power feed from said power supply means to said processing circuit when said monitor means has sensed any abnormality, and for restarting said power feed to said processing circuit after a predetermined time period has lapsed since the stop of said power feed.

9. A radiation image diagnosis apparatus, comprising:

   radiation detection means having a plurality of detection elements each of which converts radiation into an electric signal;
   a bias power source which applies a bias voltage to the detection elements;
   a processing circuit which processes the electric signals outputted from said detection elements;
   power supply means for supplying power to said processing circuit;
   monitor means for monitoring at least one of the power feed from said bias power source to said detection elements and the power feed from said power supply means to said processing circuit;
   control means for stopping said power feed from said power supply means to said processing circuit when said monitor means has sensed any abnormality; and
notification means for notifying an operator of the fact that said power feed to said processing circuit has been stopped.

10. A radiation image diagnosis apparatus, comprising:

   radiation detection means having a plurality of detection elements each of which converts radiation into an electric signal;
   a bias power source which applies a bias voltage to the detection elements;
   a processing circuit which processes the electric signals outputted from said detection elements;
   power supply means for supplying power to said processing circuit;
   monitor means for monitoring at least one of the power feed from said bias power source to said detection elements and the power feed from said power supply means to said processing circuit;
   control means for stopping said power feed from said power supply means to said processing circuit when said monitor means has sensed any abnormality; and
   storage means for storing the fact that said power feed to said processing circuit has been stopped.

11. A radiation image diagnosis apparatus, comprising:

   radiation detection means having a plurality of detection elements each of which converts radiation into an electric signal;
   a processing circuit which processes the electric signals outputted from the detection elements;
   power supply means for supplying power to said processing circuit;
   monitor means for monitoring a status of said electric signal which is outputted from at least one of said plurality of detection elements; and
   control means for temporarily stopping the power feed from said power supply means to said processing circuit when said monitor means has sensed any abnormality in said electric signal.

*FIG. 1*

*FIG. 2*

GAMMA-RAY

FIG. 3

FIG. 4

GAMMA-RAY

FIG. 5

GAMMA-RAY

## FIG. 6

## FIG. 7

FIG. 8

FIG. 9

INTERACTIVE
OPERATION UNIT — 21

DATA PROCESSING/
CONTROL UNIT — 24

DATA ACQUISITION
UNIT — 22

CAMERA HEAD
CONTROL UNIT — 33

POWER
DISTRIBUTION
SWITCH — 29

POWER
SOURCE
MONITOR — 28

POWER
SOURCES — 30 (31)

SUBJECT

## FIG. 10

## FIG. 11A

## FIG. 11B

FIG. 12

## FIG. 13

## FIG. 14

# EP 1 343 024 A1

## INTERNATIONAL SEARCH REPORT

| | International application No. |
|---|---|
| | PCT/JP00/08250 |

**A. CLASSIFICATION OF SUBJECT MATTER**
Int.Cl$^7$ G01T1/161, 1/24, 1/17

According to International Patent Classification (IPC) or to both national classification and IPC

**B. FIELDS SEARCHED**

Minimum documentation searched (classification system followed by classification symbols)
Int.Cl$^7$ G01T1/16-7/ 12

Documentation searched other than minimum documentation to the extent that such documents are included in the fields searched

| | | | | |
|---|---|---|---|---|
| Jitsuyo Shinan Koho | 1926-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, 7-248395, A (Toshiba Corporation), 26 September, 1995 (26.09.95), Full text; all drawings (Family: none) | 1-11 |
| A | JP, 6-123779, A (Horiba Ltd.), 06 May, 1994 (06.05.94), Full text; all drawings (Family: none) | 1-11 |
| A | JP, 2000-250664, A (Fujitsu Limited), 14 September, 2000 (14.09.00), Full text; all drawings (Family: none) | 1-11 |

☐ Further documents are listed in the continuation of Box C.    ☐ See patent family annex.

| | |
|---|---|
| *   Special categories of cited documents: <br> "A"   document defining the general state of the art which is not considered to be of particular relevance <br> "E"   earlier document but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| 20 February, 2000 (20.02.00) | 06 March, 2001 (06.03.01) |

| Name and mailing address of the ISA/ <br> Japanese Patent Office | Authorized officer |
|---|---|
| Facsimile No. | Telephone No. |

Form PCT/ISA/210 (second sheet) (July 1992)